# EUROPEAN PATENT APPLICATION

(11) **EP 1 062 876 A1**
(43) Date of publication of application: **27.12.2000**
(21) Application number: 99200544.7
(22) Date of filing: 25.02.1999
(51) Int. Cl.: A23J 3/10, A23L 1/305, A61K 38/17, A61K 38/01

(54) **Caseinoglycomacropeptides as calcification agent**

(71) Applicant: SOCIETE DES PRODUITS NESTLE S.A., 1800 Vevey (CH)
(72) Inventor: The designation of the inventor has not yet been filed
(74) Representative: Vuille, Roman

(57) **Abstract**

Use of caseinoglycomacropeptides (CGMPs) in a bioavailable form for the preparation of a composition intended for inhibiting bone resorption and/or favoring calcification in mammals; and composition, nutritional formula or food supplement prepared thereof.

## Description

### Field of the invention

This invention relates to the use of caseinoglycomacropeptides (CGMPs) in a bioavailable form for the preparation of a composition intended for inhibiting bone resorption and favoring calcification in mammals.

### Background of the invention

CGMP is a sialylated macropeptide which is formed by the action of the rennet or pepsin on kappa-casein from the milk of mammals.

It is known that CGMP can adhere to various types of surfaces, e.g. polystyrene (J.R. Neeser et al., Infection and Immunity, **56**, 12, 3201-3208, 1988) and saliva-coated hydroxyapatite (J.R. Neeser et al., Oral Microbiol Immunol., **9**, 193-201, 1994), thereby preventing bacterial adherence to pellicle-coated teeth. It can thus protect against dental caries by inhibiting the adherence of cariogenic bacteria which induce caries.

More recently B. Chabance et al. (Biochimie **80**, 155-165, 1998) showed that many peptides derived from α-, β- or κ-caseins, including the k-caseinoglycopeptide, were detected in stomach and blood which indicates that CGMP can cross the intestinal barrier.

Nevertheless, there is no indication in the prior art on the use of CGMP for inhibiting bone resorption or favoring bone calcification in mammals.

### Summary of the invention

Accordingly, in one aspect this invention relates to the use of caseinoglycomacropeptides (CGMPs) in a bioavailable form for the preparation of a composition intended for inhibiting bone resorption and/or favoring calcification in mammals.

In fact, it has been surprisingly found that these glycopeptides can bind to bone mineral and have an inhibitory activity on bone resorption (as shown in examples 2); they also exhibit a stimulatory activity on the metabolic activity of bone-forming cells (as shown in example 3).

Caseinoglycomacropeptides (CGMPs) may then be used in any suitable form, including salts of calcium or sodium, for example. The amount of CGMPs in the composition may be of about 0.01% to about 10% by weight on dry matter, and preferably about 0.5% to about 5% by weight on dry matter.

In another aspect this invention provides a nutritional formula intended for inhibiting bone resorption and/or favoring calcification which contains a source of protein and at least 0.01% by weight based on dry matter of CGMP in a bioavailable form. As a source of protein, dietary proteins are preferably used in any suitable form. Milk proteins, whey proteins and soy proteins are particularly preferred. The composition may also contain a source of carbohydrates and a source of fat.

This invention also relates to a food supplement and/or a food product intended for inhibiting bone resorption and/or favoring calcification, which contains at least 0.01% by weight based on dry matter of CGMP in a bioavailable form.

### Detailed Description of the Invention

In the specification, the term "bioavailable" means that CGMP(s) and/or its subcomponents and/or its bioactive hydrolytic products could cross the intestinal barrier and be recovered in an active form in the blood.

According to one aspect of the invention, the CGMPs used for the preparation of said composition may be in any suitable form of CGMP, for example salts of sodium or calcium.

CGMP can be obtained by an ion-exchange treatment of a liquid lactic raw material containing CGMP. Suitable starting materials of lactic origin may include for example:
- the product of the hydrolysis with rennet of a native casein obtained by acidic precipitation of skimmed milk with a mineral acid or acidifying ferments, optionally with addition of calcium ions,
- the hydrolysis product of a caseinate with rennet,
- a sweet whey obtained after separation of casein coagulated with rennet,
- a sweet whey or such a whey demineralized, for example, by electrodialysis and/or ion exchange and/or reverse osmosis,
- a concentrate of sweet whey,
- a concentrate of whey proteins obtained by ultrafiltration and diafiltration of sweet whey.
- mother liquors of the crystallization of lactose from a sweet whey,
- a permeate of ultrafiltration of a sweet whey.

The method preferably used to prepare CGMP is described in WO 98/53702 and consists in the decationization of the liquid raw material, such that the pH has a value of 1 to 4.5, bringing the said liquid into contact with a weak anionic resin of hydrophobic matrix, predominantly in alkaline form up to a stabilized pH, then separation of the resin and the liquid product which is recovered, and desorption of CGMP from the resin.

In one aspect a nutritional formula comprising a source of protein and at least CGMP can be prepared. Dietary proteins are preferably used as a source of protein. The dietary proteins may be any suitable dietary protein; for example animal proteins (such as milk proteins, meat proteins and egg proteins); vegetable proteins (such as soy protein, wheat protein, rice protein, and pea protein); mixtures of free amino acids; or combinations thereof. Milk proteins such as casein, whey proteins and soy proteins are particularly preferred. The composition may also contain a source of carbohydrates and a source of fat.

If the nutritional formula includes a fat source, the fat source preferably provides about 5% to about 55% of the energy of the nutritional formula; for example about 20% to about 50% of the energy. The lipids making up the fat source may be any suitable fat or fat mixture. Vegetable fats are particularly suitable; for example soy oil, palm oil, coconut oil, safflower oil, sunflower oil, corn oil, canola oil, lecithins, and the like. Animal fats such as milk fats may also be added if desired.

A source of carbohydrate may be added to the nutritional formula. It preferably provides about 40% to about 80% of the energy of the nutritional composition. Any suitable carbohydrates may be used, for example sucrose, lactose, glucose, fructose, corn syrup solids, and maltodextrins, and mixtures thereof.

Dietary fibre may also be added if desired. If used, it preferably comprises up to about 5% of the energy of the nutritional formula. The dietary fibre may be from any suitable origin, including for example soy, pea, oat, pectin, guar gum, gum arabic, and fructooligosaccharides.

Suitable vitamins and minerals may be included in the nutritional formula in an amount to meet the appropriate guidelines.

One or more food grade emulsifiers may be incorporated into the nutritional formula if desired; for example diacetyl tartaric acid esters of mono- and di-glycerides, lecithin and mono- and di-glycerides. Similarly suitable salts and stabilisers may be included.

The nutritional formula intended for preventing bone resorption and favoring calcium absorption is preferably enterally administrable; for example in the form of a powder, a liquid concentrate, or a ready-to-drink beverage. If it is desired to produce a powdered nutritional formula, the homogenized mixture is transferred to a suitable drying apparatus such as a spray drier or freeze drier and converted to powder.

In another embodiment, a usual food product may be enriched with CGMP. For example, a fermented milk, a yogurt, a fresh cheese, a renneted milk, a confectionery bar, breakfast cereal flakes or bars, drinks, milk powders, soy-based products, non-milk fermented products or nutritional supplements for clinical nutrition. Then, the amount of CGMP added is preferably of at least about 0.01% by weight.

This calcification agent may also be incorporated in article of confectionery, for example a sweet, sweetened beverages.

The following examples are given by way of illustration only and in no way should be construed as limiting the subject matter of the present application. Percentages and parts are by weight unless otherwise indicated.

### Examples

### Example 1: Preparation of CGMP

A bovine sweet whey is used which has been previously concentrated to 17% dry matter, and then demineralized by electrodialysis, freed of cation on a strong cationic resin column, freed of anion on a weak anionic resin column and spray-dried in a drying tower, of the composition indicated below:

| | % |
|---|---|
| Proteins (GMP included) | 11.7 |
| Lactose | 81.7 |
| Ash | 1 |
| Lipids | 1 |
| Water | balance for 100 |

This demineralized whey powder is solubilized in deionized water. After cation removal the solution has an initial pH of 3.8. In the preceding plant, 392 kg of this solution are treated at the temperature of 8°C, while stirring it in the reactor in the presence of 23 kg of weak anionic resin of hydrophobic matrix based on polystyrene (IMAC HP 661®, Rohm & Haas, regenerated in OH- form) for 4 h. Stabilization of the pH at 4.89 indicates the end of the reaction. The liquid is then drawn off and the resin is recovered as above.

After concentration of the liquid to 45% dry matter by evaporation, the concentrate is spray-dried in a drying tower.

Analysis of the concentrate by HPLC shows that the reaction has removed 89% of the starting GMP. Moreover, the powder contains 9.1% of whey proteins, which corresponds to a yield of 90% of the whey proteins.

To recover the GMP, the resin is washed successively with deionized water, with 30 l of an aqueous solution at 0.5% HCl and with 30 l of deionized water, and then the GMP is eluted with twice 40 l of aqueous solution at 2% Ca(OH)2 and the rinsing is carried out with 30 l of deionized water. After having combined the eluate and rinsing volumes, the whole is concentrated to a volume of 25 l by ultrafiltration with a membrane having a nominal cut-off of 3000 daltons, and then the retentate is freeze-dried and 900 g of GMP are obtained, corresponding to a yield of 80% relative to the starting GMP.

### Example 2: Resorption of dentine by disaggregated osteoclasts isolated from rat bone.

### Materials and methods

Osteoclasts were isolated from femura and tibia dissected from 1 to 2 days old rats. The bone were curretted with a scalpel in MEM containing Earle's salt with 15 mM NaHCO3 and 10% foetal bovine serum (FBS). The cell suspension was added on ivory slices and the cells were allowed to adhere for 40 min incubation time. After washing off non-adhering cells, individual slices were placed into 48-well tissue culture dishes and incubated for 24 hours with medium containing 10% FBS and different concentrations of CGMP as prepared in example 1. The cells were fixed and stained for tartrate resistent acid phosphatase (TRAP), a specific marker for osteoclasts and subsequently counted. The cells were then removed by ultrasound. After washing and drying, the slices were gold-sputtered and the pits scored using a tangential light source. In one experiment, ivory slices were pretreated with CGMP for 1 hr. Thereafter, the CGMP-containing media were removed and the osteoclasts allowed to adhere to the mineral surface of the dentine.

CGMP and bovine serum albumin dissolved in alpha-MEM at a concentration of 10 mg/ml bound about 0.3 to 0.4 mM and about 0.2mM Ca, respectively, as measured with a calcium-electrode.

### Results

The results are given in Table 1. First, the effect of CGMP on the formation of osteoclasts was assessed with the "Osteoclast Resorption Assay". CGMP was added at different concentrations to cells comprising a mixture of pre- and mature rat osteoclasts, osteoblasts that had been allowed to adhere to the surface of dentine slices (4.0x4.0x0.1)mm, prior to adding the test substance. Except where marked, all cultures were carried out in the presence of 10% fetal bovine serum (FBS). As control protein, bovine serum albumin was used. Cells were cultured for 24 hours. Thereafter the number of tartrate resistant acid phosphatase-positive multinucleated cells (TRAP+ MNC found on 16 individually cultured dentine slices, utilizing two different pools of osteoclasts (row 1). After scoring the osteoclasts, the same were removed by ultrasound. Thereafter the slices were washed, air-dried and mounted onto metal disks where they were sputter coated with gold. Using a light microscope equiped with a tangential light source, excavations were enumerated by two different persons.
The data shown the percentage of pits scored on 16 individual cultured dentine slices utilizing 2 different pools of osteoclasts (row 2). Row 3 depicts the ratio of pits and TRAP+MNC shown in rows 1 and 2.

Then, the effect of CGMP was investigated when the protein was added to the medium after the osteoclasts had attached to the mineral surface. As shown in Table1, row 1, CGMP had only a weak effect on the number of osteoclasts. The significant decrease at 10 mg/ml seems not to be specific since albumin had a similar effect.
By contrast, the number of pits per ivory was decreased by CGMP and increased by albumin at a concentration of 10 mg/ml (Table1, row 2). Calculating from row 1 and 2, the number of pits per osteoclast demonstrates that CGMP inhibits the bone resorbing activity of dissaggregated osteoclasts at a concentration of 1 and 10 mg/ml highly significantly (Table 1, row 3), whereas albumin stimulates this activity.

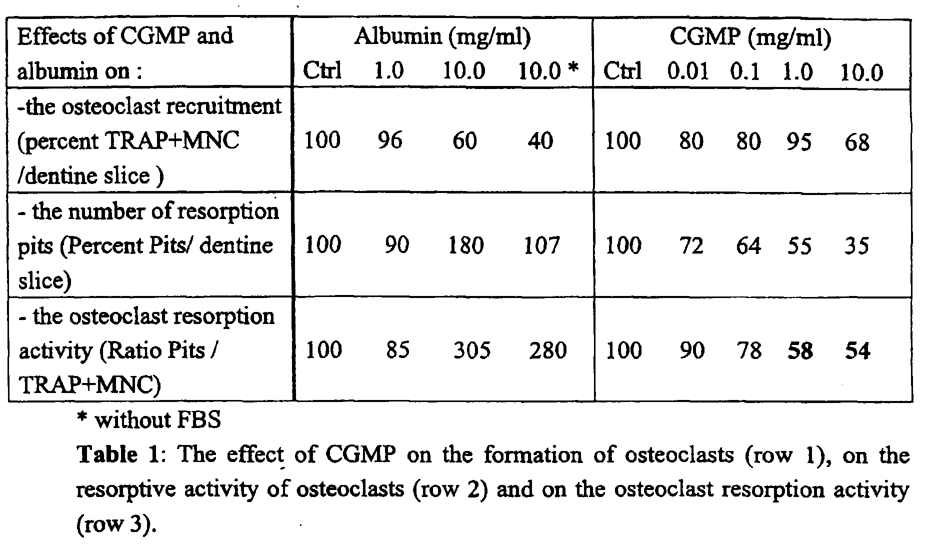

### Example 3: Effects of CGMP on osteosarcoma cell viability/proliferation

### Materials and Methods

*1. Cells*
   - **HOS58 human osteosarcoma cells**. This cell line was characterized as a highly differentiated osteoblast-like osteosarcoma cell line (Kern et al., 1990, Calcif. Tiss. Int. **46**, Suppl.2, A54), which under appropriate conditions can be stimulated to a highly differentiated phenotype, as indicated by vitamin D receptors expression and mineralization.
   - **TE 85 human osteosarcoma cells**. This cell line was obtained from ATCC (CRL1543), an osteogenic osteosarcoma cell line derived from a human 13y female. It can be grown to either high or low density, depending on medium composition. These cells are rapidly growing osteoblast-like cells with characteristics of poorly differentiated early osteoblasts.
*2. Colorimetric assay to quantify cell proliferation and viability with MTT-tetrazolium.*

A modified method of T.Mosman's assay system (J. Immunol. Methods (1983) **65**, 55-63) was used to measure cell proliferation in growing cells and the viability of resting cells in growth-arrested cultures. At the end of treatment periods, either 48 or 72 hours, cells were incubated for 4 hours with 10 µl MTT-tetrazolium stock solution (Sigma, 5 mg/ml PBS) per 100 µl of cell culture medium in a CO₂ incubator at 37°C. After the reaction period, the medium-substrate mixture was carefully removed by suction and the dark-blue formazan crystals were dissolved overnight at 37°C in 100 µl of 20% sodium dodecylsulfate (SDS). The optical density of the dye solution was measured at 550 nm.

### Results

Effects of the CGMP on cell viability/proliferation was performed starting either from high cell density cultures, which had been first grown for 1 day at 10% FCS, followed by 2 days at 1% FCS and finally for 3 days in serum-free, BSA-supplemented medium with both agents or at lower initial cell density for 1 day with 10% FCS, followed immediately by treatment with the CGMP in serum-free, BSA-supplemented medium (table 2).

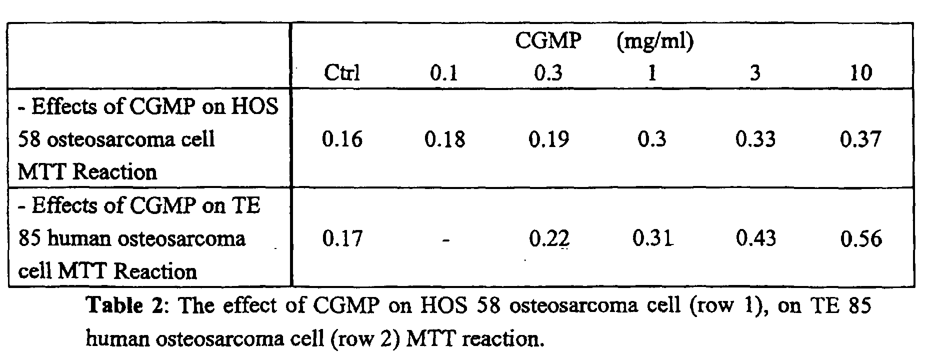

The results from both series of experiments showed, that the overall effects were similar at both culture conditions. CGMP induced a dose-dependent increase in MU reactions with TE 85 cells being the most responsive cell type towards both agents. As shown in table 2, a dose of 10 mg/ml of CGMP maximally stimulated the metabolic activity of TE 85 by more than 3-fold. The same pattern was also observed in HOS 58 cells (Table 2).

### Example 4 : Fermented milk containing CGMPs

A traditional fermented milk with 1-4 % fats was prepared as follow: After standardizing whole milk, low fat milk or a mixture of both, 0.05% by weight of CGMPs as prepared in example 1 are added. The whole was pasteurized in a plate exchanger, the liquid was cooled to the fermentation temperature, a thermophilic or mesophilic lactic ferment was added and incubation was carried out until a pH of < 5 was obtained.

The subsequent operations of filling and sealing pots took place in a conventional manner.

Additions of 0.1 %, 0.25 % and 0.5% by weight of CGMPs have also been tested for the preparation of such fermented milk which are particularly intended for favoring bone calcification and/or inhibiting bone resorption.

### Example 5 : Fermented and gelled milk enriched in probiotic bacteria containing CGMP

Fermented and gelled milks were prepared which were enriched in probiotic bacteria. 89.3 parts milk containing fat were mixed with 3.7 parts of skimmed milk powder and about 0.05 by weight of CGMP as prepared in example 1, then the mixture was preheated to 70°C and pasteurized at 92°C/6 min, and after having been cooled to 43°C the mixture was inoculated with 2% of a common yogurt starter comprising Streptococcus thermophilus and Lactobacillus bulgaricus and with 5% of Lactobacillus johnsonii (La-1, CNCM I-1225). After conditioning in pots, fermentation was carried out at 38°C up to pH 4.6 and the pots were then cooled to 6°C.

Fermented milks enriched in probiotic bacteria have also been prepared adding the following amounts of CGMP: 0.1 %, 0.25 % and 0.5% by weight. Products thus obtained have shown to have an increased stimulatory effect on the metabolic activity of bone forming cells and an inhibitory activity on bone resorption.

### Example 6 : Fermented and gelled milk enriched in probiotic bacteria containing CGMP

Fermented and gelled milks are prepared as described in example 5, wherein Lactobacillus johnsonii strain is replaced by Lactobacillus acidophilus La-10 (Nestec collection, Lausanne, Switzerland).

### Example 7: enteral composition containing CGMP

An enteral composition with an energy density of 6.3 kJ/ml and 8% (p/v) of proteins is prepared from "low temperature" skimmed milk powder, i.e. skimmed milk dried under controlled thermal conditions.

20 kg of low temperature skimmed milk powder are dispersed in 100 kg of demineralized water at a temperature of about 50-55°C. This dispersion is microfiltered by passing demineralized water through until 600 kg of permeate have been eliminated. The retentate is then further concentrated to around 60 kg, which represents a dry matter content of 21% with a protein content, based on dry matter, of 82%.

To prepare the enteral composition, 2.3 kg of liquid retentate are mixed at 55°C with 600 g of maltodextrine, 200 g of sucrose, 20.3 g of Tri-K citrate H₂O, 9.2 g of MgCl₂6H₂O, 5.8 g of NaCl and about 0.5 to 1 % by weight of CGMP as prepared in example 1.

After the ingredients have dissolved in the retentate, demineralized water is added to a total weight of the dispersion of 4.7 kg. The pH is then adjusted to 6.8, after which 300 g of fatty phase are introduced, the total weight of the dispersion being 5 kg.

After homogenization and sterilization, the product has an agreeable sugary taste and it has an inhibitory effect on bone resorption and stimulates calcification.

### Example 8 : Cereal bar containing CGMP

In order to prepare an expanded starting product, barley, wheat, corn or oat flour is treated in a twin-screw extruder for about 15 seconds at a screw speed of about 350 r.p.m. in the presence of approximately 12% of water. After the treatment, the expanded product leaves the extruder in the form of 2 to 3 mm long granules which are then dried for 20 minutes at 100°C. The product thus obtained has a cellular structure and has the following composition:
- Edible fibers: 31%
- Proteins: 21%
- Glucides: 37.5%
- Lipids: 6.5%
- Ash: 2.4%
- Water: 1.6%

The expanded product is incorporated in a bar intended for favoring bone calcification and inhibiting bone resorption, which have the following composition:
- Expanded product: 39.4%
- Oat flakes: 16.7%
- Sorbitol: 8.4%
- Fructose: 8.5%
- Apple cubes: 6.1%
- Rice crispies: 4.1%
- Gelatine: 4.0%
- Abricot powder: 2.5%
- Palm oil: 3.0%
- CGMP as prepared in example: 2.5%
- Water: 4.8%

### Example 9 : Food supplement containing CGMP

A culture of the strain Lactobacillus johnsonii (La-1, CNCM I-1225) of human origin, is mixed with CGMP as prepared in example 1 and spray dried according to the process given in EP0818529 so as to obtain a food supplement containing an amount of about 5% by weight of CGMP.

The obtained powder may be used as a food supplement. Breakfast cereals, milk product or another food products may then be sprinkled with this powder containing CGMP.

### Example 10 : Food supplement containing CGMP

A food supplement is prepared as described in example 9, except that Lactobacillus johnsonii is replaced by Lactobacillus acidophilus, La-10 (Nestec collection, Lausanne, Switzerland) or a mixture of the two strains.

## Claims

1. Use of caseinoglycomacropeptides (CGMPs) in a bioavailable form for the preparation of a composition intended for inhibiting bone resorption and/or favoring calcification in mammals.

2. Use of CGMPs according to claim 1, wherein bioavailable forms of CGMPs contain at least CGMPs themselves and/or their subcomponents and/or their bioactive hydrolytic products.

3. Use of CGMPs according to claims 1 or 2, wherein CGMPs are in the form of calcium or sodium salts.

4. Use of CGMPs according to any of claims 1 to 3, in which CGMPs are in an amount of about 0.0 1% to about 10% by weight on dry matter.

5. A nutritional formula intended for inhibiting bone resorption and/or calcification in mammals, which contains a source of protein and at least 0.01% by weight on dry matter of CGMP.

6. A nutritional formula according to claim 5, which contains a source of protein providing 7 to 25% of the total energy, a source of carbohydrates which provides 28 to 66% of the total energy, a source of lipids which provides 25 to 60% of the total energy, minerals and vitamins to meet daily requirements.

7. A composition or a food supplement intended for inhibiting bone resorption and/or favoring calcification in mammals, which contains at least about 0.01% by weight of CGMP in a bioavailable form.

8. A composition or a food supplement according to claim 7, wherein bioavailable forms of CGMPs contain at least CGMPs themselves and/or their subcomponents and/or their bioactive hydrolytic products.

9. A composition or a food supplement according to claims 7 or 8, wherein CGMPs are in the form of calcium or sodium salts.
